# EUROPEAN PATENT APPLICATION

(11) **EP 2 873 670 A1**
(43) Date of publication of application: **20.05.2015**
(21) Application number: 13192945.7
(22) Date of filing: 14.11.2013
(51) Int. Cl.: C07F 7/00, A61K 49/00, A61K 51/00, A61P 35/00

(54) **Bis azainositol zirconium complexes for X-ray imaging**

(71) Applicant: Bayer Pharma Aktiengesellschaft, 13353 Berlin (DE)
(72) Inventor: Berger, Markus, 12167 Berlin (DE); Suelzle, Detlev, 13465 Berlin (DE); Frenzel, Thomas, 12247 Berlin (DE); Pietsch, Hubertus, 14532 Kleinmachnow (DE); Jost, Gregor, 10318 Berlin (DE); Neis, Christian, 66646 Alsweiler (DE); Hegetschweiler, Kaspar, 66123 Saarbrücken (DE)
(74) Representative: BIP Patents

(57) **Abstract**

The present invention relates to a new class of trinuclear Zirconium complexes comprising of general formula (I) 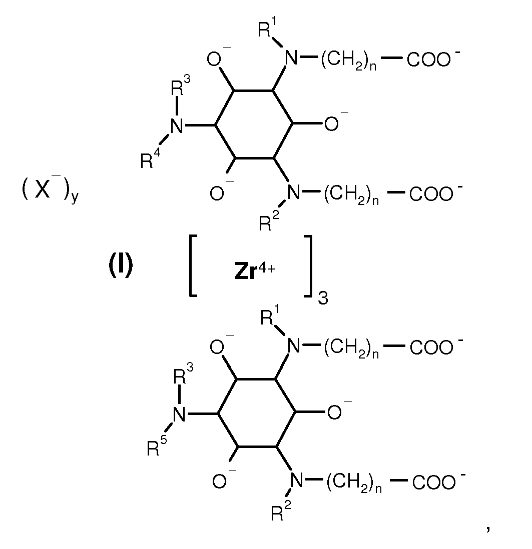 in which the substituents at the cyclohexyl rings exhibit an all-cis configuration, and in which R¹, R², R³, R⁴, R⁵, m, n, y and X are as defined in the claims, to methods of preparing said compounds, and to the use of said compounds as X-ray contrast agents.

## Description

### Field of the invention

The present invention relates to new bis azainositol Zirconium complexes comprising new azainositol ligands, to methods of preparing said compounds and to the use of said compounds as X-ray contrast agents.

### Background of the invention

The synthesis and co-ordination chemistry of 1,3,5-triamino-1,3,5-trideoxy-*cis*-inositol (taci) and a multitude of derivatives of this cyclohexane-based polyamino-polyalcohol have widely been examined in the past by Hegetschweiler et al. (Chem. Soc. Rev. 1999, 28, 239). Among other things, the ability of taci and of the hexa-*N,N',N"*-methylated ligand tdci to form trinuclear complexes of the composition [M₃(H₋₃taci)₂]³⁺ and [M₃(H₋₃tdci)₂]³⁺, respectively, with a unique, sandwich-type cage structure in the presence of heavy metals M^{III} like Bi^{III} or a series of lanthanides was described (Chem. Soc. Rev. 1999, 28, 239; Inorg. Chem. 1993, 32, 2699; Inorg. Chem. 1998, 37, 6698). But, due to their moderate solubility in water and their deficient thermodynamic stability, these complexes proved not to be suitable for *in vivo* applications.

Complex formation of taci with more than 30 metal ions has been investigated and the metal cations can be divided into five categories according to the adopted coordination mode that was verified by crystal structure analyses (Chem. Soc. Rev. 1999, 28, 239). Although this classification helpfully reviews the coordination properties of taci, it has to be pointed out that multiple metals do not fit into the presented scheme. As a consequence, a prediction of the preferred coordination mode for metals that have not been described so far is often ambiguous. In addition to that, it was demonstrated that modifications at the ligand backbone can have a strong impact on the coordination behavior (Inorg. Chem. 1997, 36, 4121). This is not only reflected in the structural characteristics of the metal complexes but can often lead to unpredictable changes in their thermodynamic and/or kinetic complex stability, water solubility and other physicochemical parameters. The ability to form trinuclear Zirconium complexes with a sandwich-type cage structure has never been reported before for any taci derivative.

Moreover, the synthesis of mononuclear carboxylic acid derived taci metal complexes has been reported by Laboratorien Hausmann AG, St. Gallen, CH in DE 40 28 139 A1 and WO 92/04056 A1 for iron^{III} and gadolinium^{III}. A possible application of its mononuclear, radioactive metal complexes as radiopharmaceuticals was also claimed.

All-*cis*-1,3,5-triamino-2,4,6-cyclohexane triol derivatives, their use and methods for their preparation were also described by Laboratorien Hausmann AG in EP, A, 190 676.

Byk Gulden Lomberg Chemische Fabrik GmbH described taci based transition metal complexes for magnetic resonance diagnostics in WO 91/10454.

Nycomed AS in WO 90/08138 described heterocyclic chelating agents for the preparation of diagnostic and therapeutic agents for magnetic resonance imaging, scintigraphy, ultrasound imaging, radiotherapy and heavy metal detoxification.

The formation of trinuclear iron^{III} complexes was suggested by G. Welti (Dissertation, Zürich 1998**)** for an acetate and by A. Egli (Dissertation, Zürich 1994**)** for a 2-hydroxybenzyl derivative of taci. G. Welti also described the synthesis of rhenium^{V} and rhenium^{VII} complexes of acetate derived ligands based on taci with a M₁L₁ stoichiometry.

D. P. Taylor & G. R. Choppin (Inorg. Chim. Acta 2007, 360, 3712) described the formation of mononuclear lanthanide complexes with similar derived ligands and determined a pM value of 6.0 for complexes with europium^{III} which means that the stability under physiological conditions is even lower than that of europium^{III} complexes of unmodified taci.

General Electric Company described nanoparticle compositions in WO 2012/080290 comprising metal oxides including Zirconium oxide and their potential use as contrast agents in medical imaging techniques such as X-ray. This relates to an earlier report on dextrin-stabilized aquasols of Zirconium and Zirconium dioxides (Zirconotrast, Hafnotrast) in the context of their biokinetics (Environmental Research 1979, 18, 127) and to US 5326552 X-ray imaging compositions based on Zirconium oxide nano particles.

The use of Zirconium based x-ray contrast agent will be especially effective in contrast enhanced x-ray mammography performed with conventional projection imaging or with more advanced tomographic imaging methods like thomosynthesis or potentially dedicated breast computed tomography. In contrast to MRI based mammography the x-ray procedures today are usually performed without contrast agents. The use of contrast agents has the potential to increase the diagnostic performance of mammography procedures significantly. However, the contrast agent available for intravascular administration today are not efficient in the x-ray energy range used for mammography.

Mammography imaging units operates with x-ray tube voltages in the range of 35 to 49 kV. The respective x-ray energy spectra enable high imaging contrast to detect breast tumors/calcifications at lowest radiation dose. However, the signal efficacy of the well-established iodinated x-ray contrast agents is rather low in this energy range. Zirconium based contrast agents offers a significantly higher x-ray absorption (quantified by the mass-attenuation coefficient) for the x-ray energies used in mammography. Thus, contrast enhanced x-ray mammography will become feasible. The use of Zirconium will also allow for energy subtraction imaging in digital mammography as described by Lawaczeck et al. (Investigative Radiology 2003, 38, 602).

In summary, the state of the art described above consists of physiologically stable or thermodynamically instable heavy metal complexes which hold a metal that is not suitable for the x-ray energy range used in mammography.
The aim of the present invention was to provide sufficiently stable, water soluble and well tolerated Zirconium complexes with a high metal content for use as X-ray contrast agents in diagnostic imaging, especially in the x-ray energy range used in mammography or in the energy subtraction imaging mode.

This aim was achieved by the provision of the compounds of the present invention. It has been found, that tri-*N,N',N"*-substituted derivatives of taci (L) effectively form new complexes with Zirconium of a M₃L₂ stoichiometry which grants a high Zirconium content of > 20% for the compounds of the present invention. Surprisingly, it was observed that these complexes show a very high stability in aqueous solution for this type of stoichiometry under heat sterilization conditions and have an excellent tolerability in experimental animals as well as a high *in vivo* stability.

After intravenous injection the compounds of the present invention are excreted fast and quantitatively via the kidneys, comparable to the well established triiodinated X-ray contrast agents.

The invention of suitable new bis-azainositol Zirconium complexes enables for the first time the practical use of this compound class as X-ray contrast agents in diagnostic imaging.

### Summary of the invention

The present invention describes a new class of trinuclear Zirconium complexes comprising two hexadentate azainositol carboxylic acid ligands, methods for their preparation and their use as low kV X-ray contrast agents.

### Detailed description of the invention

In a first aspect, the invention is directed to compounds of the general formula (I), 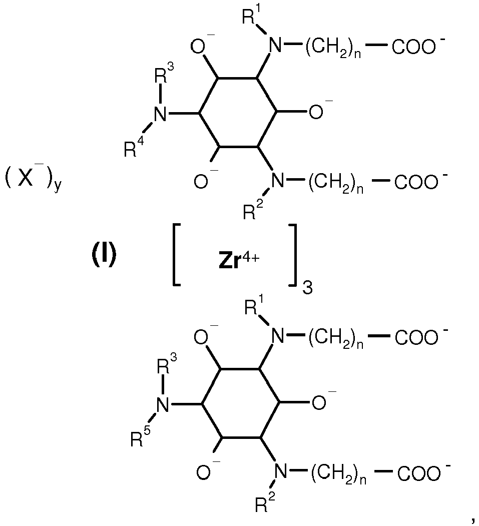 wherein
the substituents at the cyclohexyl rings exhibit an all-cis configuration;
R¹, R² and R³ are independently from each other H or methyl;
R⁴ and R⁵ are independently from each other H, CH₃, CH₂CH₃, CH₂CH₂CH₃, CH₂CH₂OH CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), CH(CH₂OH)(CH₂COO⁻), CH(CH₂CH₂OH)(COO)⁻ or CH(CH₂OH)(COO⁻);
m is 1 or 2;
n is 1 or 2;
y is 0, 1 or 2;
   and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

The present invention includes all possible stereoisomers of the compounds of the present invention, as single stereoisomers, or as any mixture of said stereoisomers, e.g. R- or S- isomers, in any ratio. Isolation of a single stereoisomer, e.g. a single enantiomer or a single diastereomer, of a compound of the present invention may be achieved by any suitable state of the art method, such as chromatography, especially chiral chromatography, for example. Compounds containing chiral centers may be used as racemic mixture or as an enantiomerically enriched mixture or as a diastereomeric mixture or as a diastereomerically enriched mixture, and an individual stereoisomer may be used alone.

The present invention also relates to useful forms of the compounds as disclosed herein, such as hydrates, solvates, salts, in particular pharmaceutically acceptable salts, and co-precipitates.

Trinuclear Zirconium complexes of the general formula (I), which are charged at physiological pH, can be neutralized by addition of suitable, physiologically biocompatible counter ions.

Suitable anions are the anions of inorganic acids, such as, for example, hydrochloric acid, phosphoric acid and sulfuric acid, as well as the anions of organic acids, such as, for example, acetic acid, citric acid, aspartic acid, glutamic acid, among others can be used.

The compounds of the present invention can exist in the form of a salt. Said salt may be any salt, either an organic or inorganic salt, particularly any pharmaceutically acceptable organic or inorganic salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

Pharmaceutically acceptable salts of the compounds according to the invention include salts of mineral acids, carboxylic acids and sulfonic acids, for example salts of hydrochloric acid, sulfuric acid, phosphoric acid, methanesulfonic acid, ethanesulfonic acid, toluenesulfonic acid, benzenesulfonic acid, acetic acid, propionic acid, lactic acid, tartaric acid, malic acid, citric acid, fumaric acid, maleic acid and benzoic acid.

The present invention includes all possible salts of the compounds of the present invention as single salts, or as any mixture of said salts, in any ratio.

For the manufacture of diagnostic agents, for example the administration to human or animal subjects, the compounds of general formula (I) will conveniently be formulated together with pharmaceutical carriers or excipient. The contrast media of the invention may conveniently contain pharmaceutical formulation aids, for example stabilizers, antioxidants, pH adjusting agents, flavors, and the like. They may be formulated for parenteral or enteral administration or for direct administration into body cavities. For example, parenteral formulations contain a sterile solution or suspension in a concentration range from 100 to 400 mg Zirconium/mL, especially 150 to 300 mg Zirconium/mL of the new azainositol heavy metal clusters according to this invention. Thus the media of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen and oxygen, such as ²H (deuterium), ³H (tritium), ¹¹C, ¹³C, ¹⁴C, ¹⁵N, ¹⁷O and ¹⁸O, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

Where the plural form of the word compounds, salts, polymorphs, hydrates, solvates and the like, is used herein, this is taken to mean also a single compound, salt, polymorph, isomer, hydrate, solvate or the like.

In accordance with a second embodiment of the first aspect, the present invention covers compounds of general formula (I), *supra,* wherein :
the substituents at the cyclohexyl rings exhibit an all-cis configuration;
R¹, R² and R³ are independently from each other H or methyl;
R⁴ and R⁵ are independently from each other H, CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), CH(CH₂OH)(CH₂COO⁻), CH(CH₂CH₂OH)(COO⁻) or CH(CH₂OH)(COO⁻);
m is 1 or 2;
n is 1 or 2;
y is 0, 1 or 2;
   and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

In accordance with a third embodiment of the first aspect, the present invention covers compounds of general formula (I), *supra,* wherein :
the substituents at the cyclohexyl rings exhibit an all-cis configuration;
R¹, R² and R³ are independently from each other H or methyl;
R⁴ and R⁵ are independently from each other H, CH₂CH₂OH, CH₂CH₂CH₂OH, CH₂CH(OH)CH₂OH, CH₂CH(OH)CH(OH)CH₂OH, or (CH₂)ₘCOO⁻;
m is 1 or 2;
n is 1 or 2;
y is 0, 1 or 2;
   and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

In accordance with a fourth embodiment of the first aspect, the present invention covers compounds of general formula (I), *supra,* wherin :
the substituents at the cyclohexyl rings exhibit an all-cis configuration;
R¹, R² and R³ are independently from each other H or methyl;
R⁴ and R⁵ are independently from each other H or (CH₂)ₘCOO⁻;
m is 1 or 2;
n is 1 or 2;
y is 0, 1 or 2;
   and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

Another embodiment of the first aspect are compounds of formula (I) selected from the group consisting of:
[Zr₃(H₋₃tacita)₂] = Bis[µ₃-2,2',2"-({[1*R*-(1α,2α,3α,4α,5α,6α)]-2,4,6-trihydroxylato-1κ²*O*²*O*⁶, 2κ²*O*²*O*⁴,3κ²*O*⁴*O*⁶-cyclohexane-1,3,5-triyl}triimino-1κ*N*¹,2κ*N*³,3κ*N*⁵)triacetato-1κ*O*,2κ*O*',3κ*O*"]trizirconium(IV) 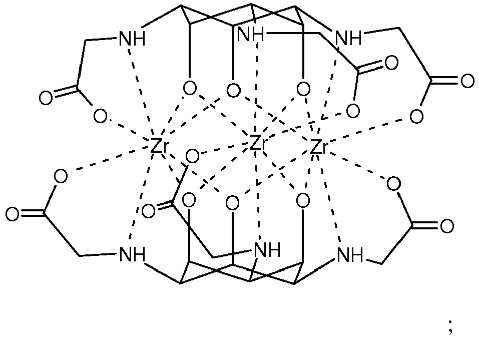
[Zr₃(H₋₃tacitp)₂] = Bis[µ₃-3,3',3"-({[1*R*-(1α,2α,3α,4α,5α,6α)]-2,4,6-trihydroxylato-1κ²*O*²*O*⁶, 2κ²*O*²*O*⁴,3κ²*O*⁴*O*⁶-cyclohexane-1,3,5-triyl}triimino-1κ*N*¹,2κ*N*³,3κ*N*⁵)tripropanoato-1κ*O*,2κ*O*',3κ*O*"]trizirconium(IV) 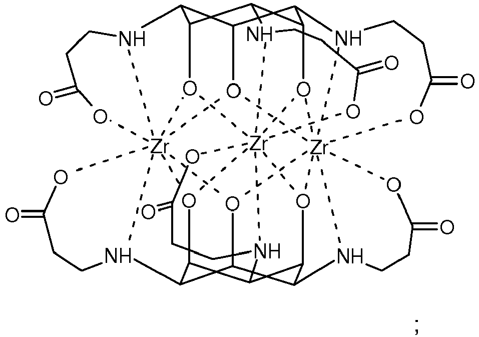
[Zr₃(H₋₃macitp)₂] = Bis[µ₃-3,3',3"-({[1*R*-(1α,2α,3α,4α,5α,6α)]-2,4,6-trihydroxylato-1κ²*O*²*O*⁶, 2κ²*O*²*O*⁴,3κ²*O*⁴*O*⁶-cyclohexane-1,3,5-triyl}tris{methylimino-1κ*N*¹,2κ*N*³,3κ*N*⁵})tripropanoato-1κ*O*,2κ*O*',3κ*O*"]trizirconium(IV) 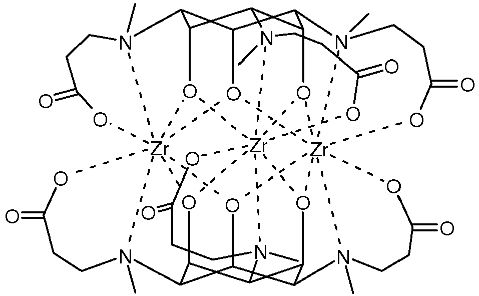
[Zr₃(H₋₃macitp)(H₋₃macidp)OH] = Hydroxido-3K*O*-[µ₃-3,3',3"-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-cyclohexane-1,3,5-triyl}tris{methylimino-1κ*N*¹,2κ*N*³,3κ*N*⁵})tripropanoato-1κ*O*,2κ*O*',3κ*O*"][µ₃-3,3'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[methylamino-3κ*N*⁵]cyclohexane-1,3-diyl}bis-{methylimino-1κ*N*¹,2κ*N*³})dipropanoato-1κ*O*,2κ*O*']trizirconium(IV) 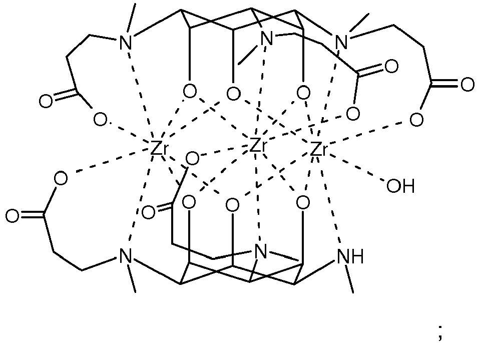
[Zr₃(H₋₃tacidpma)₂] = Bis[µ₃-3,3'-({[1*R*-(1α,2α,3α,4α,5α,6α)]-5-[(carboxylato-3κ*O*-methyl) amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹, 2κ*N*³)dipropanoato-1κ*O*,2κ*O*']trizirconium(IV) 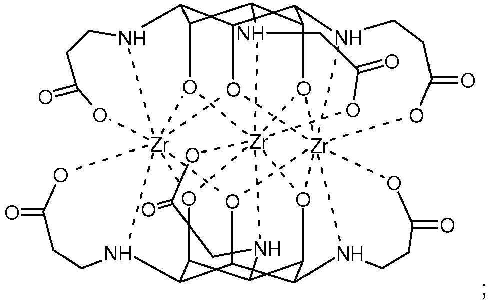 and
[Zr₃(H₋₃tacidamp)₂] = Bis[µ₃-2,2'-({[1*R*-(1α,2α,3α,4α,5α,6α)]-5-[(2-carboxylato-3κ*O-*ethyl)amino-3κ*N*⁵]-2,4,6-trihydroxylato-1κ²*O*²*O*⁶,2κ*O*²*O*⁴,3κ*O*⁴*O*⁶-cyclohexane-1,3-diyl}diimino-1κ*N*¹,2κ*N*³)diacetato-1κ*O*,2κ*O*']trizirconium(IV)

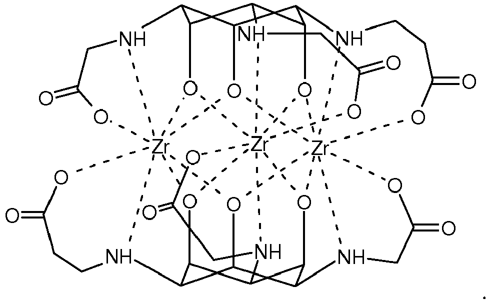

In a second aspect, the invention is directed to the process for the preparation of the compounds of the general formula (I).

In one embodiment of the second aspect, the invention is directed to the process for the preparation of the compounds of the general formula (I) *supra,* from carboxylic acids of the general formula (II), 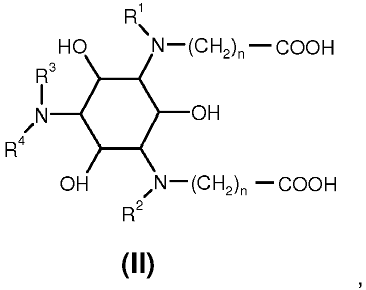 wherein
the substituents at the cyclohexyl ring exhibit an all-cis configuration;
R¹, R² and R³ are independently from each other H or methyl;
R⁴ is H, CH₃, CH₂CH₃, CH₂CH₂CH₃, CH₂CH₂OH CH₂CH₂CH₂OH, CH₂CH(OH)CH₂OH, CH₂CH(OH)CH(OH)CH₂OH, CH(CH₂OH)₂, (CH₂)ₘCOOH, CH(CH₂OH)(CH(OH)CH₂OH), CH(CH₂OH)(CH₂COOH), CH(CH₂CH₂OH)(COOH), CH(CH₂OH)(COOH);
m is 1 or 2;
n is 1 or 2;
or a protonated species, a deprotonated species, a hydrate, a solvate, or a salt thereof, and a Zirconium (IV) halogenide,
wherein
halogenide is either chloride or bromide,
or hydrates thereof,
or,
Zirconium(IV) oxychloride,
or hydrates thereof,

in aqueous solution under elevated temperatures, using conventional heating methods or microwave irradiation, ranging from 80°C to 180°C, in a pH range of 1 to 7, preferably at 110° to 160 °C in a pH range of 1 to 4.

In a second embodiment of the secend aspect, the present invention also relates to a method of preparing a compound of general formula (I) *supra,* said method comprising the step of allowing an intermediate compound of general formula (II) 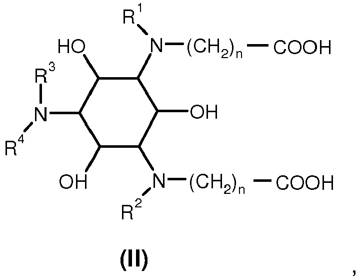 in which
the substituents at the cyclohexyl ring exhibit an all-cis configuration;
R¹, R² and R³ are independently from each other H or methyl;
R⁴ is H, CH₃, CH₂CH₃, CH₂CH₂CH₃, CH₂CH₂OH CH₂CH₂CH₂OH, CH₂CH(OH)CH₂OH, CH₂CH(OH)CH(OH)CH₂OH, CH(CH₂OH)₂, (CH₂)ₘCOOH, CH(CH₂OH)(CH(OH)CH₂OH), CH(CH₂OH)(CH₂COOH), CH(CH₂CH₂OH)(COOH), CH(CH₂OH)(COOH);
m is 1 or 2;
n is 1 or 2;
or a protonated species, a deprotonated species, a hydrate, a solvate, or a salt thereof, to react with
a Zirconium (IV) halogenide,
in which
halogenide is either chloride or bromide,
or hydrates thereof,
or, to react with
Zirconium(IV) oxychloride,
or hydrates thereof,
thereby giving a compound of general formula (I) 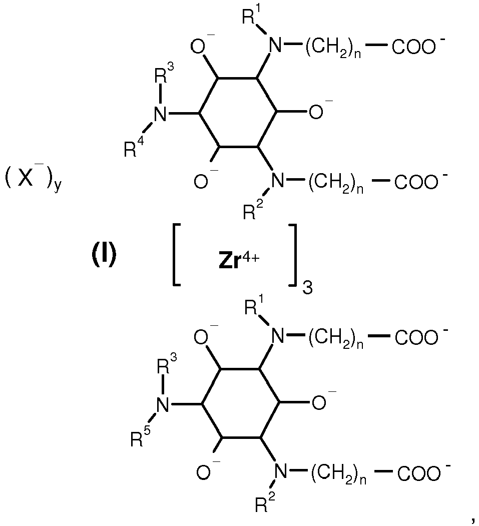 in which the substituents at the cyclohexyl rings exhibit an all-cis configuration, and in which R¹, R², R³, R⁴, R⁵, m, n, y and X are as defined for the compound of general formula *(I) supra.*

In a further aspect, the invention is directed to compounds of general formula (I) for the manufacture of diagnostic agents, especially of X-ray diagnostic agents, for administration to humans or animals.

Another aspect of the invention is the use of the compounds of general formula (I) for the manufacture of diagnostic agents.

Another aspect of the invention is the use of a compound of general formula (I) for diagnostic imaging, especially as X-ray diagnostic agent.

Another aspect of the invention is the use of a compound of general formula (I) for use in the diagnosis of a disease.

Another aspect of the invention is the use of a compound of general formula (I) in the diagnosis of a disease.

Another aspect of the invention is the use of a compound of general formula (I) in the diagnosis of a cancer, especially in the diagnosis of breast cancer.

Another aspect of the invention is the use of a compound of general formula (I) as diagnostic agent.

### General synthesis of compounds of the invention

The present invention provides carboxylic acid derived ligands based on 1,3,5-triamino-1,3,5-trideoxy-cis-inositol (taci) that can readily form trinuclear, highly stable Zirconium(IV) complexes with high water solubility. The tri-O-benzylated taci derivative all-cis-2,4,6-tris(benzyloxy)-1,3,5-cyclohexanetriamine **(tbca)** was used as starting material throughout. It can be prepared as reported by Bartholomä et al. (Chem. Eur. J. 2010, 16, 3326). The ligand tbca can be alkylated in the presence of bases like cesium carbonate or N,N-diisopropylethylamine with *tert*-butyl-halogenoacetate in organic solvents like THF or dichloromethane. The ligand **tacita** was synthesized according to G. Welti (Dissertation, Zürich 1998**)** using the tri-*O*-benzylated **taci** derivative **tbca** as starting material which was alkylated in the reaction with the sterically demanding agents *N,N*-diisopropylethylamine and *tert*-butyl-bromoacetate (Scheme 1). The protecting groups were removed in boiling hydrochloric acid and pure H₃tacita was isolated by precipitation at pH 5.5.

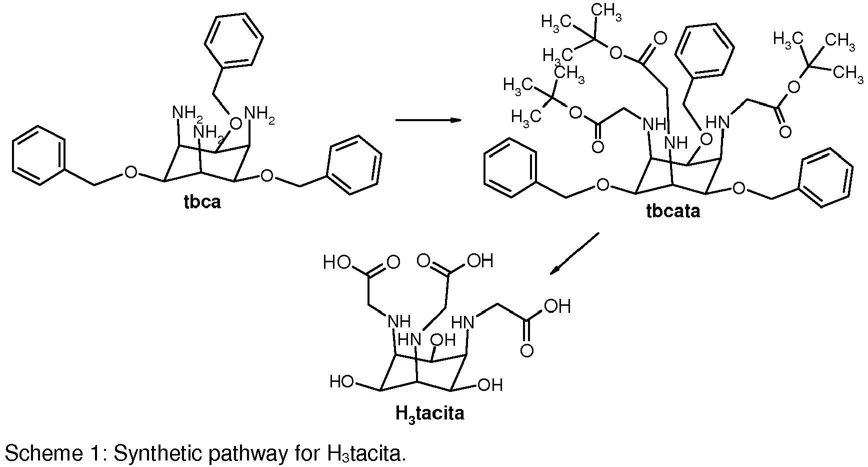

The synthesis of the tri-*N,N',N"-*propionic acid derivative **(tacitp)** was first of all reported by Laboratorien Hausmann AG, St. Gallen, CH , in DE 40 28 139 A1, 1992**.** Herein, we describe a modified procedure in which the ligand taci dissolved in methanol reacts with acrylonitrile in a first step (Scheme 2). The intermediate was finally hydrolyzed to the tricarboxylic acid in alkaline solution (25 % sodium hydroxide). The pure ligand was conveniently obtained in the hydrochloride form by cation exchange chromatography.

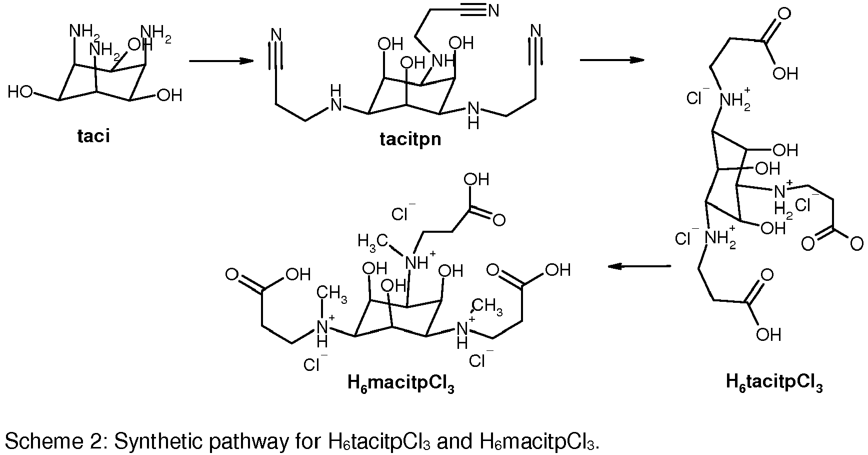

Introduction of additional methyl groups was obtained by catalytic hydrogenation of aqueous solutions of the ligands in the presence of formaldehyde. The ligands were eventually purified and isolated in their hydrochloride form by cation exchange chromatography.

The statistically formed monoalkylation **(tbcama)** and dialkylation **(tbcada)** products are purified by preparative HPLC or by chromatography on amino phase silica gel. Depending on the equivalents of alkylating agent used, the twofold derived ligand **tbcada** or the one fold derived ligand **tbcama** (Scheme 3) can be obtained as main product.

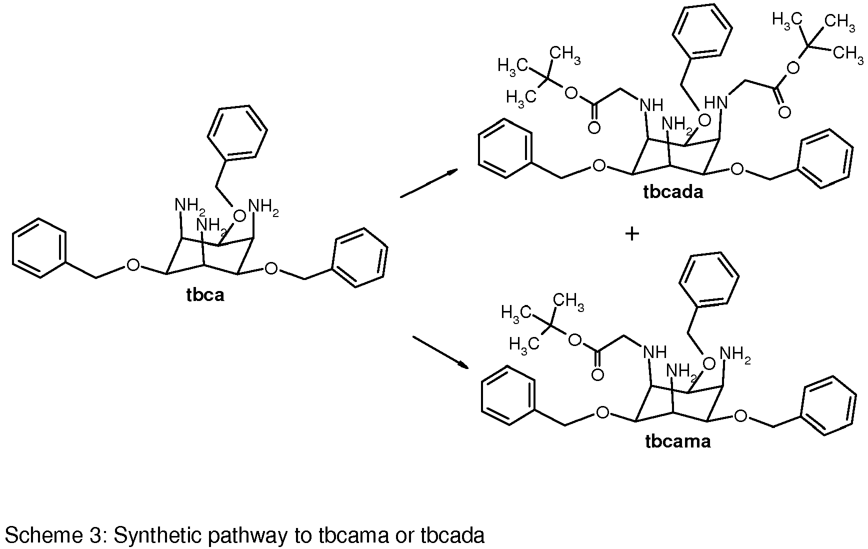

Introduction of the third amino substituent at the primary amine can be accomplished by reductive amination procedure or Michael addition of acrylic acid derivatives like acrylonitrile or acrylic esters. Aldehydes or ketones can be used in combination with a suitable reducing agent like 5-ethyl-2-methylpyridine borane complex (Tetrahedron Lett. 2008, 49, 5152), sodium triacetoxyborohydride or sodium cyanoborohydride (Comprehensive Organic Synthesis, Pergamon: Oxford 1991, 8, 25). In case of glycolaldehyde and D,L-glyceraldehyde the available dimers can be used to deliver the subsequent **tbcadamx** derivatives (Scheme 4). A third possibility to introduce a third substituent at the primary amine is the alkylation with halogenoalkanes in the presence of bases. The pure ligands are conveniently obtained in the hydrochloride form by cation exchange chromatography after removal of the protecting groups under strong acidic conditions.

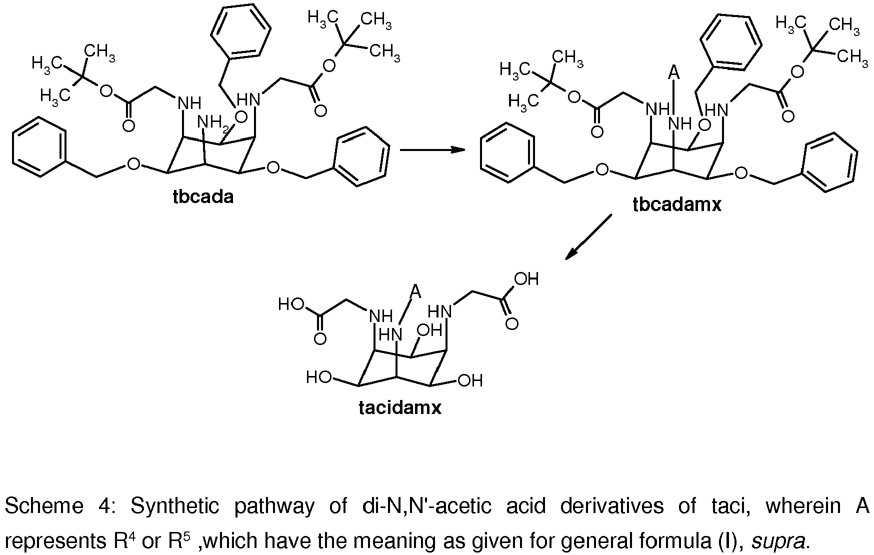

Starting from **tbcama** the ligand **tacidpma** with one acetic acid and two propionic acid as amino substituents can be prepared analogously by treatment with acrylonitrile or acrylic ester and subsequent acidic deprotection of the **tbcadpma** intermediate (Scheme 5).

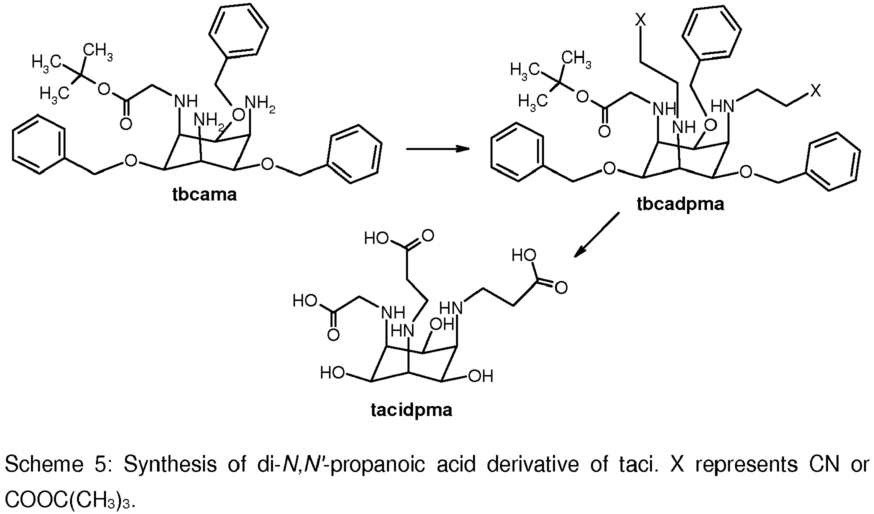

For the synthesis of further di-*N,N'*-propanoic acid derivatives of taci, the building block **tbcadpn** can be prepared from **tbca** using stoichiometric amounts of acrylonitrile. Introduction of the third amino substituent different from already present substituents to the intermediate **tbcadpmx** can analogously to **tbcadamx** be introduced at the primary amino group by reductive amination of appropriate aldehydes or ketones or by halogen-alkanes in the presence of base. (Scheme 6). The pure **tacidpmx** ligands can be obtained by simultaneous removal of the protecting groups and nitrile or ester hydrolysis under strong acidic conditions.

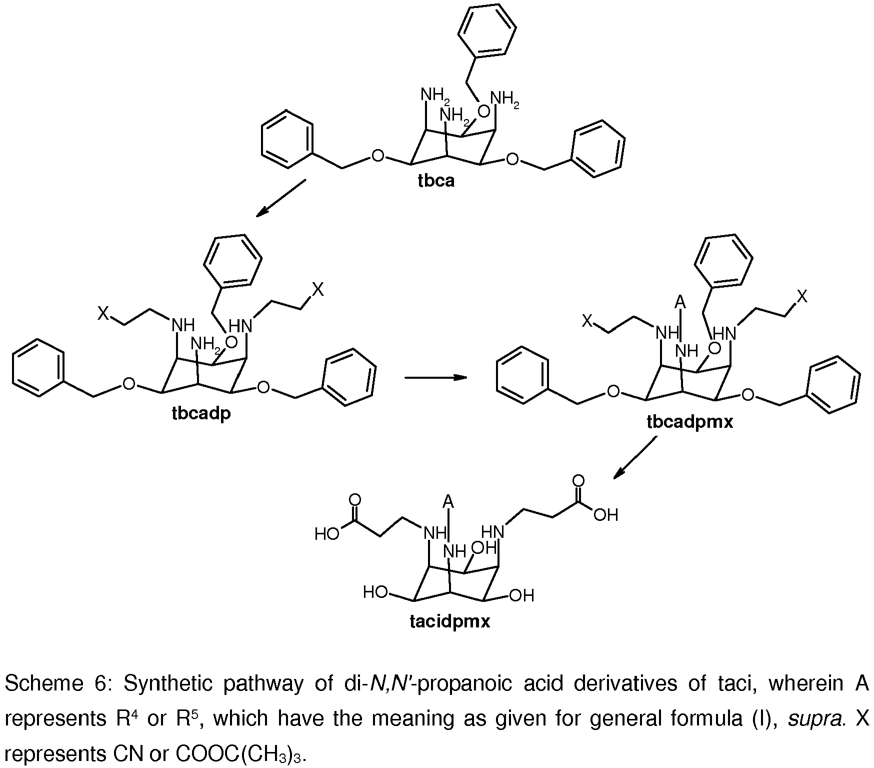

Trinuclear Zirconium(IV) complexes of the accordingly prepared ligands can be synthesized by adding stoichiometric amounts or minor excess of Zirconium salts like Zirconium(IV)chloride to aqueous solutions of the ligand (Scheme 7). The reaction mixtures can be heated by conventional methods or by microwave irradiation at a pH range from 1 to 7 for at least 15 minutes at a temperature range from 110 °C to 160 °C under pressure.

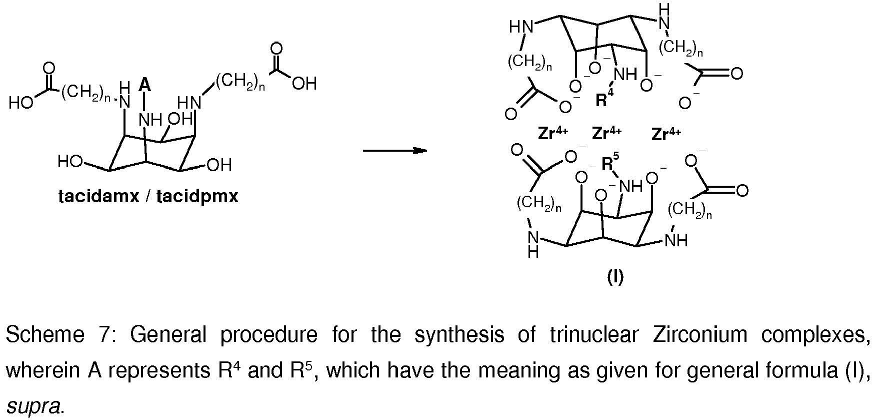

Isolation and purification of the desired complexes can be achieved by preparative HPLC, ultrafiltration or crystallization methods.

### Description of the Figures

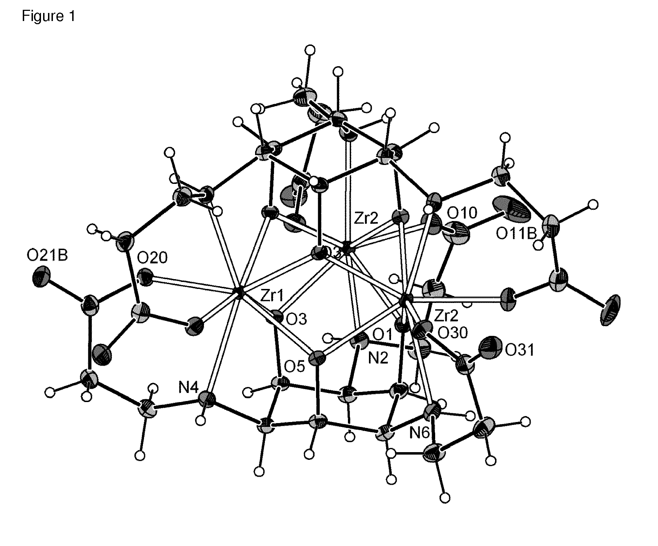

Figure 1: Crystal structure of of [Zr₃(H₋₃tacitp)₂] (Example 2). The displacement ellipsoids are drawn at the 50 % probability level; H(-N) hydrogen atoms are shown as spheres of arbitrary size; H(-C) hydrogen atoms are omitted for clarity.

### Experimental Part

**Abbreviations**

| | |
|---|---|
| br. | broad signal (in NMR data) |
| DAD | diode array detector |
| DMSO | dimethylsulfoxide |
| ELSD | evaporative light scattering detector |
| ESI | electrospray ionisation |
| HPLC | high performance liquid chromatography |
| MS | mass spectrometry |
| m | multiplet |
| NMR | nuclear magnetic resonance spectroscopy: chemical shifts (δ) are given in ppm. |
| rt | room temperature |
| s | singlet |
| t | triplet |
| THF | tetrahydrofuran |
| UPLC | ultra performance liquid chromatography |

### Materials and Instrumentation

The chemicals used for the synthetic work were of reagent grade quality and were used as obtained. Dowex 50 W-X2 (100-200 mesh, H+ form) was from Sigma-Aldrich, the mixed bed ion exchange resins Amberlite MB-6113 from Merck. The starting materials 1,3,5-triamino-1,3,5-trideoxy-*cis*-inositol (= taci) (Ghisletta M., Jalett H.-P., Gerfin T., Gramlich V., Hegetschweiler K. Helv. Chim. Acta 1992, 75, 2233) and all-cis-2,4,6-tris(benzyloxy)-1,3,5-cyclohexanetriamine (= tbca) (Bartholomä, M.; Gisbrecht, S.; Stucky, S.; Neis, C.; Morgenstern, B.; Hegetschweiler, K. Chem. Eur. J. 2010, 16, 3326) were prepared as described in the literature.

¹H and ¹³C{¹H} NMR spectra were measured in D₂O or DMSO-d₆, respectively (294 K, Bruker DRX Avance 400 MHz NMR spectrometer (*B*₀ = 9.40 T), resonance frequencies: 400.20 MHz for ¹H and 100.63 MHz for ¹³C or 300 MHz spectrometer for ¹H. Chemical shifts are given in ppm relative to sodium (trimethylsilyl)propionate-d₄ (D₂O) or tetramethylsilane (DMSO-*d₆*) as internal standards (δ = 0 ppm). The pH* of the D₂O samples was adjusted using appropriate solutions of DCI in D₂O. The term pH* refers to the direct pH-meter reading (Metrohm 713 pH meter) of the D₂O samples, using a Metrohm glass electrode with an aqueous (H₂O) Ag/AgCl-reference that was calibrated with aqueous (H₂O) buffer solutions.

Elemental analyses (C,H,N) were recorded on a LECO 900V or VARIO EL analyzer.

Examples were analyzed and characterized by the following HPLC based analytical methods to determine characteristic retention time and mass spectrum:
**Method 1: UPLC** (ACN-HCOOH):
   Instrument: Waters Acquity UPLC-MS SQD 3001; column: Acquity UPLC BEH C18 1.7 50x2.1mm; eluent A: water + 0.1% formic acid, eluent B: acetonitril; gradient: 0-1.6 minutes 1-99% B, 1.6-2.0 minutes 99% B; flow 0.8 mL/minute; temperature: 60 °C; injection: 2 µL; DAD scan: 210-400 nm; ELSD
**Method 2: UPLC** (ACN-HCOOH polar):
   Instrument: Waters Acquity UPLC-MS SQD 3001; column: Acquity UPLC BEH C18 1.7 50x2.1mm; eluent A: water + 0.1% formic acid, eluent B: acetonitril; gradient: 0-1.7 minutes 1-45% B, 1.7-2.0 minutes 45-99% B; flow 0.8 mL/minute; temperature: 60 °C; injection: 2 µL; DAD scan: 210-400 nm; ELSD

### Example 1 [Zr₃(H₋₃tacita)₂]

### Bis[µ₃-2,2',2"-({[1R-(1α,2α,3α,4α,5α,6α)]-2,4,6-trihydroxylato-1κ²O²O⁶,2κ²O²O⁴,3κ²O⁴O⁶-cyclohexane-1,3,5-triyl}triimino-1κN¹,2κN³,3κN⁵)triacetato-1κO,2κO',3κO"]trizirconium(IV)

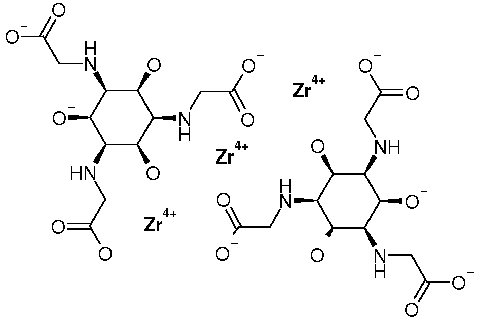

### Example 1a

### 1,3,5-Triamino-1,3,5-trideoxy-cis-inositol-tri-N,N,N"-acetic acid (H₃tacita)

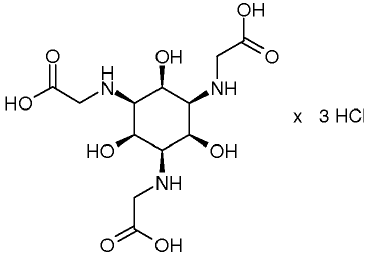

all-*cis*-2,4,6-Tris(benzyloxy)-1,3,5-cyclohexanetriamine (3.0 g, 6.7 mmol) was dissolved in dichloromethane (120 mL) and *N,N*-diisopropylethylamine (3.3 mL, 20.1 mmol) was added. tert-Butyl bromoacetate (3.4 mL, 23.5 mmol) was added dropwise to the solution which was stirred for three days at ambient temperature afterwards. The solvent was completely removed and the residue was dissolved in methanol (50 mL). After addition of 6 M hydrochloric acid (300 mL) the suspension was heated to reflux for 24 hours. The resulting solution was extracted twice with dichloromethane and the aqueous layer was evaporated to dryness. The white solid was dissolved in water (50 mL) and the pH was adjusted to 5.5 using sodium hydroxide (40 %) to get a white precipitate that was filtered off, washed with ethanol, and dried in vacuo to yield 2.5 g (92 %) of H₃tacita·3H₂O.

**¹H-NMR** (D₂O, pH* < 1) δ = 3.85 (t, 3H), 4.24 (s, 6H), 4.78 (t, 3H)ppm.
**¹³C-NMR** (D₂O, pH* < 1) δ = 45.7, 57.6, 64.4, 169.3 ppm.
**¹H-NMR** (D₂O, pH* > 13) δ = 2.57 (m, 3H), 3.32 (s, 6H), 4.12 (m, 3H) ppm.
**¹³C-NMR** (D₂O, pH* > 13) δ = 51.9, 60.5, 71.3, 182.6 ppm.
**Anal.** Calcd (%) for C₁₂H₂₁N₃O₉·3H₂O (405.36): C, 35.56; H, 6.71; N, 10.37. Found: C, 35.36; H, 6.49; N, 10.25.
**IR** (cm⁻¹): 607, 632, 679, 793, 914, 936, 978, 1012, 1133, 1214, 1283, 1328, 1371, 1404, 1574,2744,3054,3421.

### Example 1 b

### Bis[µ₃-2,2',2"-({[1R-(1α,2α,3α,4α,5α,6α)]-2,4,6-trihydroxylato-1κ²O²O⁶,2κ²O²O⁴,3κ²O⁴O⁶-cyclohexane-1,3,5-triyl}triimino-1κN¹,2κN³,3κN⁵)triacetato-1κO,2κO',3κO"] trizirconium(IV) [Zr₃(H₋₃tacita)₂]

Zirconium(IV) oxychloride octahydrate (436 mg, 1.4 mmol) was dissolved in water (50 mL). H₃tacita·4.5H₂O (390 mg, 0.9 mmol) was added and the pH was adjusted to ∼ 2.5 (1 M sodium hydroxide). The suspension was heated to reflux for 20 hours. The reaction mixture was filtered and to the filtrate was added mixed bed ion exchange resin Amberlite MB-6113 until the resin kept its blue color. The resin was filtered off and the filtrate was lyophilized to yield: 81 mg (16 %) [Zr₃(H₋₃tacita)₂]·8H₂O.

### Alternative procedure:

### [Zr₃(H₋₃tacita)₂]

Zirconium(IV) oxychloride octahydrate (436 mg, 1.4 mmol) was dissolved in water (50 mL). H₃tacita·4.5H₂O (390 mg, 0.9 mmol) was added and the pH was adjusted to ∼ 2.5 (1 M sodium hydroxide). The suspension was heated to reflux for 20 hours. Remaining solids were filtered off and the solution was ultra-filtrated through a 500 da membrane while dilution of the retentate was repeated 3 times by addition of desalted water. The retentate was collected and lyophilized to yield 185 mg (37 %) of [Zr₃(H₋₃tacita)₂]·8H₂O.

**¹H-NMR** (400 MHz, D₂O) δ = 3.68 - 3.75 (m, 6H), 3.91 - 3.95 (m , 6H), 4.10 - 4.20 (m, 6H), 4.91 (m, 1 H), 5.05 (m, 3H), 5.18 (m, 1 H) ppm.
**¹³C-NMR** (100 MHz, D₂O) δ = 52.3 , 62.5 , 62.8, 77.5, 184.6, 184.7 ppm.
**Anal.** Calcd (%) for C₂₄H₃₀N₆O₁₈Zr_{3·}8H₂O (1108.32): C, 26.01; H, 4.18; N, 7.58. Found: C, 25.67; H, 3.53; N, 7.20.
**IR** (cm⁻¹): 615, 647, 715, 816, 916, 957, 1016, 1087, 1113, 1163, 1302, 1346, 1505, 1615, 1981, 2052, 2164, 2324, 2938, 3161.
**MS** (ES⁺): *m*/*z* = 482.9 {[Zr₃(H₋₃tacita)₂]+2H}²⁺, 965.8 {[Zr₃(H₋₃tacidpma)₂]+H}⁺.
**MS** (ES-): *m*/*z* = 962.8 {[Zr₃(H₋₃tacidpma)₂]-H}⁻.

### Example 2 [Zr₃(H₋₃tacitp)₂]

### Bis[µ₃-3,3',3"-({[1R-(1α,2α,3α,4α,5α,6α)]-2,4,6-trihydroxylato-1κ²O²O⁶,2κ²O²O⁴,3κ²O⁴O⁶-cyclohexane-1,3,5-triyl}triimino-1κN¹,2κN³,3κN⁵)tripropanoato-1κO,2κO',3κO"] trizirconium(IV)

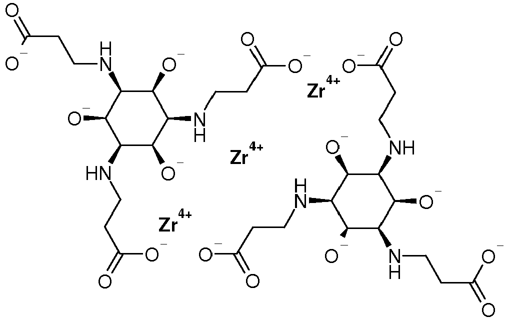

### Example 2a

### 1,3,5-Triamino-1,3,5-trideoxy-cis-inositol-tri-N,N',N"-propionitrile (tacitpn)

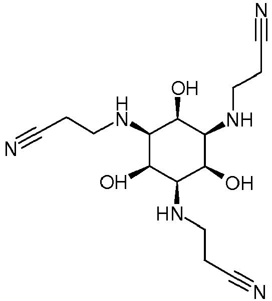

Taci (2.0 g, 11.3 mmol) was dissolved in methanol (100 mL) and acrylonitrile (7.4 mL, 0.11 mol) was added. The solution was stirred for 24 hours at ambient temperature. The solvent was removed, the residue washed successively with diethyl ether and hexane and the white solid was dried in vacuo to yield 3.9 g (97 %) of tacitpn·0.2H₂O·0.5CH₃OH.

**¹H-NMR** (400 MHz, D₂O) δ = 2.72 (m, 9H), 3.03 (t, 6H), 4.23 (t, 3H) ppm.
**¹³C-NMR** (101 MHz, D₂O) δ = 20.5, 43.4, 60.1, 72.0, 123.2 ppm.
**Anal.** Calcd (%) for C₁₅H₂₄N₆O₃·0.2H₂O·0.5MeOH (356.01): C, 52.29; H, 7.47; N, 23.61. Found: C, 52.23; H, 7.23; N, 23.40.
**IR** (cm⁻¹): 602, 754, 843, 902, 1072, 1113, 1252, 1352, 1425, 1987, 2067, 2248, 2924, 3103, 3268.
**MS** (ES⁺): *m*/*z* = 337.5 {tacitpn+H}⁺.
**MS** (ES-): *m*/*z* = 335.6 {tacitpn-H}⁻.

### Example 2b

### 1,3,5-Triamino-1,3,5-trideoxy-cis-inositol-tri-N,N',N"-propionic acid trihydrochloride (H₆tacitpCl₃)

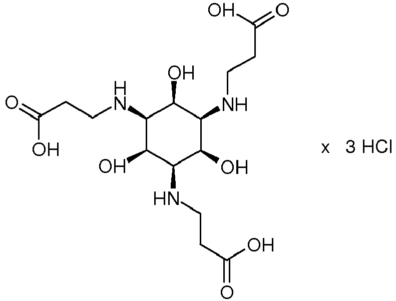

Tacitpn (3.8 g, 10.7 mmol) was dissolved in sodium hydroxide (10.3 g of a 25 % solution, 64.4 mmol) and heated to reflux for 4 hours. The solvent was removed and the residue was taken up in 1 M hydrochloric acid (5 mL) and sorbed on DOWEX 50. The column was washed with water (1 L), 0.25 M hydrochloric acid (1 L), 1 M hydrochloric acid (1 L) and the product was eluted with 3 M hydrochloric acid (1 L). The solvent was removed and the solid dried in vacuo to yield: 5.1 g (86 %) of H₃tacitp·3HCl·3H₂O.

**¹H-NMR** (400 MHz, D₂O) δ = 2.43 (t, 6H), 2.61 (m, 3H), 2.89 (t, 6H), 4.26 (m, 3H) ppm. **¹³C-NMR** (100 MHz, D₂O) δ = 40.3, 44.7, 60.5, 71.8, 184.2 ppm.
**Anal.** Calcd (%) for C₁₅H₂₇N₃O₉·3HCl·3H₂O (556.82): C, 32.36; H, 6.52; N, 7.55. Found: C, 32.56; H, 6.31; N, 7.64.
**MS** (ES⁺): *m*/*z* (%) 441.4 (100) {H₂tacitp+2Na}⁺, 394.2 (75) {H₃tacitp+H]⁺.
**MS** (ES-): *m*/*z* (%) 392.3 (100) {H₃tacitp-H}⁻.

### Example 2c

### Bis[µ₃-3,3',3"-({[1R-(1α,2α,3α,4α,5α,6α)]-2,4,6-trihydroxylato-1κ²O²O⁶, 2κ²O²O⁶,2κ²O²O⁴,3κ²O⁴O⁶-cyclohexane-1,3,5-triyl}triimino-1κN¹,2κN³,3κN⁵)tripropanoato-1κO,2κO',3κO"]trizirconium(IV) [Zr₃(H₋₃tacitp)₂]

H₃tacitp·3HCl·3H₂O (830 mg, 1.5 mmol) was dissolved in water (80 mL). 1 M sodium hydroxide (13.5 mL, 13.5 mmol) as well as Zirconium(IV) oxychloride octahydrate (805 mg, 2.5 mmol) dissolved in water (10 mL) were successively added. The pH was adjusted to ∼ 3.5 (1 M hydrochloric acid) and the suspension was heated to reflux for 3 days. The solids were filtered off and to the filtrate was added mixed bed ion exchange resin Amberlite MB-6113 until the resin kept its blue color. The resin was filtered off and the filtrate was lyophilized. Single crystals suitable for X-ray structure analysis were obtained by slow evaporation of an aqueous solution of the metal complex to yield 450 mg (46 %) of [Zr₃(H₋₃tacitp)₂]·15H₂O.

### Alternative procedure:

### [Zr₃(H₋₃tacitp)₂]

H₃tacitp·3HCl·3H₂O (165 mg, 0.3 mmol) was dissolved in water (20 mL). 1 M sodium hydroxide (2.7 mL, 2.7 mmol) as well as Zirconium(IV) oxychloride octahydrate (161 mg, 0.5 mmol) dissolved in water (5 mL) were successively added. The pH was adjusted to ∼ 3.5 (1 M hydrochloric acid) and the suspension was heated to reflux for 3 days. The solids were filtered off and to the filtrate was added mixed bed ion exchange resin Amberlite MB-6113 until the resin kept its blue color. The resin was filtered off and the filtrate was lyophilized to yield 140 mg (72 %) of [Zr₃(H₋₃tacitp)₂]·15H₂O.

**¹H-NMR** (400 MHz, D₂O) δ = 2.52 - 2.66 (m , 12H), 3.05 - 3.12 (m , 6H), 3.25 - 3.29 (m, 6H), 3.42 (m, 1 H), 3.47 (m, 1 H), 3.50 (m, 3H), 3.54 (m, 1 H), 4.81 (m, 1 H), 4.87 - 5.05 (m, 6H), 5.09 (m, 4H), 5.38 (m, 1 H) ppm .
**¹³C-NMR** (100 MHz, D₂O) δ = 36.19, 36.25 (x 2), 36.3, 45.1 (x 4), 62.2, 62.3, 62.4, 62.5, 75.8, 77.6 (x 2), 79.3, 182.3 (x 4) ppm.
**Anal.** Calcd (%) for C₃₀H₄₂N₆O₁₈Zr₃·15H₂O (1318.59): C, 27.33; H, 5.50; N, 6.37. Found: C, 26.92; H, 4.74; N, 6.18.
**IR** (cm⁻¹): 621, 811, 882, 919, 1005, 1076, 1153, 1216, 1251, 1302, 1352, 1391, 1463, 1600, 2953, 3182.
**MS** (ES⁺): *m*/*z* (%) 1071 (26) {[Zr₃(H₋₃tacitp)₂]+Na}⁺, 1049 (100) {[Zr₃(H₋₃tacitp)₂]+H}⁺.
**MS** (ES-): *m*/*z* (%) 1047 (100) {[Zr₃(H₋₃tacitp)₂]-H}⁻.

### Crystal data and structure refinement:

| | | |
|---|---|---|
| Empirical formula | C₃₀H₆₀N₆O₂₇Zr₃ | |
| Formula weight | 1210.50 | |
| Temperature | 123(2) K | |
| Wavelength | 0.71073 Å | |
| Crystal system | Monoclinic | |
| Space group | C2/c | |
| Unit cell dimensions | a = 19.2962(10) Å | α = 90°. |
| | b = 18.3041 (9) Å | β = 99.830(2)°. |
| | c = 12.0759(6) Å | γ = 90°. |
| Volume | 4202.6(4) Å³ | |
| Z | 4 | |
| Density (calculated) | 1.913 Mg/m³ | |
| Absorption coefficient | 0.836 mm⁻¹ | |
| F(000) | 2472 | |
| Crystal size | 0.48 x 0.30 x 0.12 mm³ | |
| Theta range for data collection | 1.54 to 26.37°. | |
| Index ranges | -24≤h≤24, -22≤k≤22, -15≤l≤15 | |
| Reflections collected | 50595 | |
| Independent reflections | 4299 [R(int) = 0.0221] | |
| Completeness to θ = 26.37° | 100.0 % | |
| Absorption correction | Semi-empirical from equivalents | |
| Max. and min. transmission | 0.9063 and 0.6896 | |
| Refinement method | Full-matrix least-squares on F² | |
| Data / restraints / parameters | 4299 / 9 / 343 | |
| Goodness-of-fit on F² | 1.130 | |
| Final R indices [I>2σ(I)] | R₁ = 0.0185, wR₂ = 0.0458 | |
| R indices (all data) | R₁ = 0.0191, wR₂ = 0.0463 | |
| Largest diff. peak and hole | 0.466 and -0.380 e·Å⁻³ | |

Atomic coordinates ( x 10⁴) and equivalent isotropic displacement parameters (Å²x 10³) for [Zr₃(H₋₃tacitp)₂]. U(eq) is defined as one third of the trace of the orthogonalized U^{ij} tensor.

| | x | y | z | U(eq) |
|---|---|---|---|---|
| Zr(1) | 5000 | 1426(1) | 7500 | 8(1) |
| O(1) | 4677(1) | 3395(1) | 6588(1) | 13(1) |
| C(1) | 4270(1) | 3325(1) | 5485(1) | 14(1) |
| Zr(2) | 5769(1) | 3087(1) | 6899(1) | 10(1) |
| N(2) | 3319(1) | 3329(1) | 6552(1) | 14(1) |
| C(2) | 3578(1) | 2960(1) | 5606(1) | 14(1) |
| C(4) | 4098(1) | 1731(1) | 5202(1) | 13(1) |
| N(4) | 4307(1) | 1028(1) | 5782(1) | 12(1) |
| C(3) | 3681(1) | 2159(1) | 5949(1) | 12(1) |
| O(3) | 4052(1) | 2119(1) | 7083(1) | 11(1) |
| C(5) | 4787(1) | 2106(1) | 5087(1) | 12(1) |
| O(5) | 5259(1) | 2081(1) | 6143(1) | 12(1) |
| C(6) | 4689(1) | 2902(1) | 4734(1) | 14(1) |
| N(6) | 5393(1) | 3251(1) | 4922(1) | 15(1) |
| C(7) | 3093(1) | 4095(1) | 6279(2) | 21(1) |
| C(8) | 2886(1) | 4483(1) | 7288(2) | 22(1) |
| C(9) | 3510(1) | 4667(1) | 8181(2) | 28(1) |
| O(10) | 4026(1) | 4211(1) | 8356(1) | 21(1) |
| O(11A) | 3477(2) | 5185(2) | 8843(4) | 43(1) |
| O(11B) | 3654(5) | 5325(5) | 8450(8) | 43(1) |
| C(17) | 3708(1) | 525(1) | 5818(1) | 16(1) |
| C(18) | 3951(1) | -172(1) | 6449(1) | 17(1) |
| C(19) | 4209(1) | -73(1) | 7701(1) | 15(1) |
| O(20) | 4382(1) | 586(1) | 8051(1) | 14(1) |
| O(21A) | 4283(18) | -610(10) | 8340(10) | 21(2) |
| O(21B) | 4160(20) | -550(20) | 8330(30) | 21(2) |
| C(27) | 5864(1) | 2999(1) | 4151(2) | 19(1) |
| C(28) | 6568(1) | 3391(1) | 4401(2) | 20(1) |
| C(29) | 7016(1) | 3166(1) | 5503(2) | 17(1) |
| O(30) | 6695(1) | 2866(1) | 6241(1) | 17(1) |
| O(31) | 7657(1) | 3272(1) | 5672(1) | 23(1) |
| O(1W) | 1861(1) | 4023(1) | 1067(1) | 24(1) |
| O(2W) | 2665(1) | 3624(1) | 3084(1) | 23(1) |
| O(3W) | 3091(1) | 2207(1) | 2955(1) | 28(1) |
| O(4W) | 5136(3) | 4673(3) | 3517(4) | 49(1) |
| O(5W) | 4705(3) | 5228(2) | 5892(4) | 51(1) |
| O(6W) | 5000 | 1775(1) | 2500 | 44(1) |

### Example 3 [Zr₃(H₋₃macitp)₂]

### Bis[µ₃-3,3',3"-({[1R-(1α,2α,3α,4α,5α,6α)]-2,4,6-trihydroxylato-1κ²O²O⁶, 2κ²O²O⁴,3κ²O⁴O⁶-cyclohexane-1,3,5-triyl}tris{methylimino-1κN¹,2κN³,3κN⁵})tripropanoato-1κO,2κO',3κO"]trizirconium(IV)

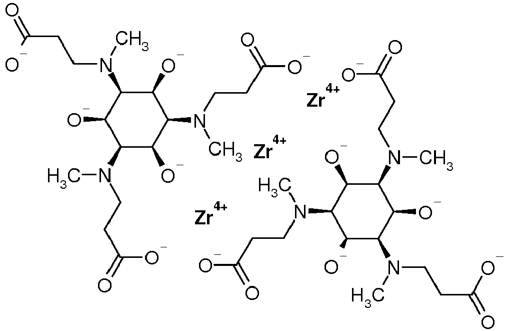

### Example 3a

### 1,3,5-Trideoxy-1,3,5-tris(methylamino)-cis-inositol-tri-N,N',N"-propionic acid trihydrochlorid (H₆macitpCl₃)

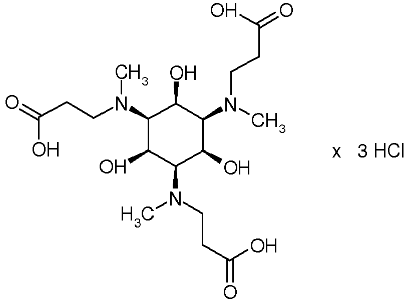

H₃tacitp·3HCl·3H₂O (400 mg, 0.7 mmol) was dissolved in a formaldehyde solution (37 %, 25 mL, 334 mmol) and palladium on charcoal (40 mg, 10 %) was added. The reaction mixture was hydrogenated in an autoclave at 50 atm H₂ for 4 days at rt. The reaction mixture was filtered off and the filtrate concentrated to dryness. The residue was dissolved twice in a 1 : 1 mixture of water and formic acid (30 mL) and evaporated to dryness again. The remaining solid was taken up in 3 M hydrochloric acid (10 mL) and sorbed on DOWEX 50. The column was washed successively with 0.5 M hydrochloric acid (1 L), 1 M hydrochloric acid (1 L) and 3 M hydrochloric acid (1 L). The 3 M fraction containing the product was evaporated to dryness and the solid was dried in vacuo to yield 320 mg (71 %) of H₃macitp·3HCl·4.5H₂O.

**¹H-NMR** (400 MHz, D₂O) δ = 3.04 (t, 6H), 3.15 (s, 9H), 3.67 (m, 3H), 3.78 (t, 6H), 5.04 (m, 3H) ppm.
**¹³C-NMR** (101 MHz, D₂O) δ = 23.6, 34.3, 45.5, 57.9, 58.6, 169.9 ppm.
**Anal.** Calcd (%) for C₁₈H₃₃N₃O₉·3HCl·4.5H₂O (625.92): C, 34.54; H, 7.25; N, 6.71. Found: C, 34.20; H, 6.86; N, 6.71.

### Example 3b

### Bis[µ₃-3,3',3"-({[1R-(1α,2α,3α,4α,5α,6α)]-2,4,6-trihydroxylato-1κ²O²O⁶, 2κ²O²O⁴,3κ²O⁴O⁶-cyclohexane-1,3,5-triyl}tris{methylimino-1κN¹,2κN³,3κN⁵})tri-propanoato-1κO,2κO',3κO"]trizirconium(IV) [Zr₃(H₋₃macitp)₂]

A solution of 1,3,5-trideoxy-1,3,5-tris(methylamino)-*cis*-inositol-tri-*N*,*N*',*N*"-propionic acid trihydrochloride (900 mg, 1.49 mmol) and Zirconium(IV)chloride (678 mg, 2.23 mmol) in water (16 mL) was the pH of each vessel was adjusted to 4.5 by addition of aqueous sodium hydroxide (1 M) The mixture was separated into 3 pressure vessels and water was added to reach a total volume of 30 mL for each. The vessels were sealed and irradiated in a microwave reactor for 20 minutes at 140°C. The combined solutions were treated with mixed bed ion exchange resins Amberlite MB-6113 until the resin kept its blue color. The filtrate was lyophilized, solved in water (300 mL) and passed through a 3000 da ultrafiltration membrane (Millipore YM3) while dilution of the retentate was repeated three times. The combined 3000 da filtrates were concentrated in vacuum while a final volume of 200 mL was lyophilized to yield 270 mg of raw product as a white solid which was purified by preparative HPLC to yield 16.8 mg of the title compound of [Zr₃(H₋₃macitp)₂] and 69.9 mg of [Zr3(H-3macitp)(H-3macidp)OH] example 4.

| | |
|---|---|
| *Column:* | C18 YMC-ODS AQ 10µm 51 x 200 mm |
| *Solvent:* | A = H₂O + 0.1% formic acid |
| | B = acetonitrile |
| *Gradient:* | 0 - 4 minute 1 % B, 4-13 minutes 1 - 25 % B |
| *Flow:* | 240 mL/minute |
| *Temperature:* | rt |
| *Detection:* | 195 nm |

**¹H-NMR** (300 MHz, D₂O): δ = 2.48 - 2.66 (m, 12H), 2.84 (d, 24H), 2.91 - 3.02 (m, 6H), 3.62 - 3.85 (m, 6H), 5.53 (br. s., 6H) ppm.
**MS** (ES⁺): *m*/*z* 1133.1 {[Zr₃(H₋₃macitp)₂]+H)⁺.
**UPLC** (ACN-HCOOH): Rt. = 0.43 min.

### Example 4 [Zr₃(H₋₃macitp)(H₋₃macidp)OH]

**Hydroxido-3κ*O*-[µ₃-3,3',3"-({[1*****R**-***(1α,2α,3α,4α,5α,6α)]-2,4,6-trihydroxylato-1κ²*O*²*O*⁶, 2κ*O*²*O*⁴,3κ²*O*⁴*O*⁶-cyclohexane-1,3,5-triyl]tris{methylimino-1κ*N*¹,2κ*N*³,3κ*N*⁵}) tri-propanoato-1κO,2κO',3κO"][µ₃-3,3'-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-5-[methylamino-3κ*N*⁵]cyclohexane-1,3-diyl}bis{methyl-imino-1κ*N*¹,2κ*N*³})dipropanonato-1κ*O*,2κ*O*']trizirconium(IV)**

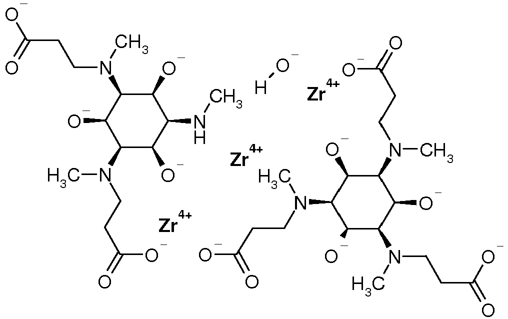

The title compound was isolated by HPLC as additional product from example 3b.

**¹H-NMR** (300 MHz, D₂O): δ = 2.47 - 2.69 (m, 12H), 2.73 - 2.76 (m, 6H), 2.77 - 2.88 (m, 14H), 2.92 - 2.98 (m, 6H), 3.18 - 3.22 (m, 1 H), 3.53 - 3.87 (m, 5H), 5.07 - 5.13 (m, 1 H), 5.40 (br. s., 1 H), 5.51 (br. s., 4H) ppm.
**MS** (ES⁺): *m*/*z* 1062.7 [Zr₃(H₋₃macitp)(H₋₃macidp)]⁺
**MS** (ES-): *m*/*z* 1078.1 {[Zr₃(H₋₃macitp)(H₋₃macidp)OH]-H]⁻.
**UPLC** (ACN-HCOOH): Rt. = 0.40 min.

### Example 5 [Zr₃(H₋₃tacidpma)₂]

### Bis[µ₃-3,3'-({[1R-(1α,2α,3α,4α,5α,6α)]-5-[(carboxylato-3κO-methyl)amino-3κN⁵]-2,4,6-trihydroxylato-1κ²O²O⁶,2κ²O²O⁴,3κ²O⁴O⁶-cyclohexane-1,3-diyl}diimino-1κN¹,2κN³) dipropanoato-1κO,2κO']trizirconium(IV)

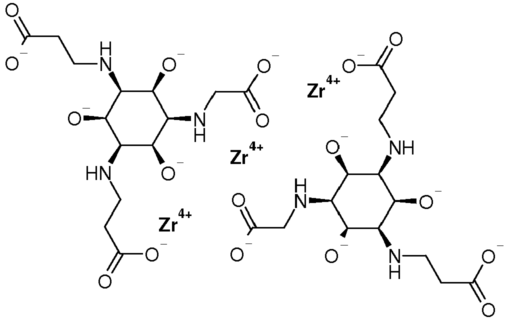

### Example 5a

### Tert-butyl 2-({[1R-(1α,2α,3α,4α,5α,6α)]-3,5-diamino-2,4,6-tris[benzyloxy]cyclohexane-1-yl}imino)acetate (tbcama)

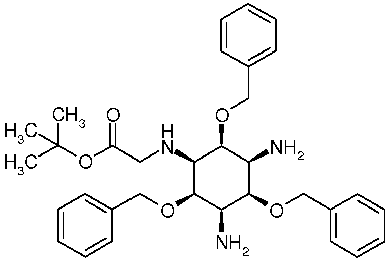

All-cis-2,4,6-tris(benzyloxy)-1,3,5-cyclohexanetriamine (2.0 g, 4.47 mmol, Chem. Eur. J., 2010, 16, 3326-3340) was dissolved in THF (72 mL) and cesium carbonate (1.60 g, 4.92 mmol) was added. *Tert*-butyl bromoacetate (1.66 g, 8.49 mmol) was added at room temperature and the mixture stirred for 20 hours. The mixture was filtered, the filtrate was concentrated under reduced pressure and the residue was purified by chromatography on amino phase silica gel (ethyl acetate in hexane, 40 to 100% then ethanol in ethyl acetate, 0 to 20%) to yield 0.95 g of the title compound (tbcama).

**¹H-NMR** (400 MHz, CDCL₃): δ = 1.47 (s, 9H), 3.08 (t, 1 H), 3.16 (t, 2H), 3.55 - 3.63 (m, 3H), 3.66 (s, 2H), 4.58 - 4.74 (m, 6H), 7.29 - 7.44 (m, 15H) ppm.
**MS** (ES⁺): *m*/*z* (%) 562.2 (53) {tbcama+H}⁺, 506.2 (100) {tbcama-^{t}Bu+H}⁺.

As a second product 0.76 g of di-*tert*-butyl 2,2'-({[1*R*-(1α,2α,3α,4α,5α,6α)]-5-amino-2,4,6-tris[benzyloxy]cyclohexane-1,3-diyl}diimino)diacetate (tbcada) was isolated.

**¹H-NMR** (300 MHz, DMSO-*d6*): δ = 1.38 (s, 18H), 2.71 (br. s., 2H), 3.26 - 3.35 (m, 6H), 3.39 - 3.43 (m, 2H), 3.50 - 3.59 (m, 2H), 4.49 - 4.69 (m, 6H), 7.20 - 7.45 (m, 15H) ppm. **¹³C-NMR** (75 MHz, DMSO-*d6*): δ = 27.6, 51.4, 51.7, 57.1, 70.2, 70.3, 74.4, 75.7, 80.3, 127.3, 127.3, 127.5, 128.2, 137.9, 138.1, 171.8 ppm.
**MS** (ES⁺): *m*/*z* (%) 676 (27) {tbcada+H)⁺, 620 (100) {tbcada-^{t}Bu+H}⁺, 564 (48) {tbcada-2×^{t}Bu+H}⁺, 474 (8) {tbcada-2×^{t}Bu-Bn+H}⁺.

### Example 5b

### 3,3'-({[1R-(1α,2α,3α,4α,5α,6α)]-5-[(Carboxymethyl)amino]-2,4,6-trihydroxy cyclohexane-1,3-diyl}diimino)dipropanoic acid trihydrochloride (H₆tacidpmaCl₃)

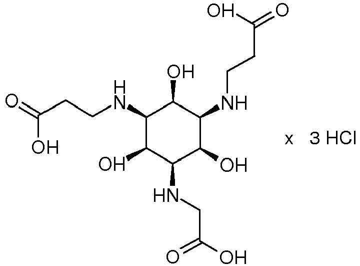

Tbcama (300 mg, 0.5 mmol) was dissolved in methanol (30 mL). Acrylonitrile (350 µL, 5.3 mmol) was added and the solution was stirred for 2 days at ambient temperature. The solvent was removed and *tert*-butyl *N-*({[1*R*-(1α,2α,3α,4α,5α,6α)]-2,4,6-tris[benzyloxy]-3,5-bis[(2-cyanoethyl)amino]-cyclohexane-1-yl}imino)acetate was obtained as crude product. The residue (350 mg) was suspended in 6 M hydrochloric acid (50 mL) and heated to reflux for 3 h. After cooling, the solution was evaporated to dryness, the remaining solid dissolved in 0.5 M hydrochloric acid (5 mL) and sorbed on DOWEX 50. The column was washed with water (0.5 L) and 0.5 M hydrochloric acid (1 L) and the product was eluted with 3 M hydrochloric acid (1.2 L). The eluant was removed and the solid dried in vacuo to yield 246 mg of H₃tacidpma·3HCl·2.5H2O.

**¹H-NMR** (400 MHz, D₂O pH*=0): δ = 2.99 (t, 4H), 3.57 (t, 4H), 3.77 (t, 2H), 3.84 (t, 1 H), 4.23 (s, 2H), 4.77 (m, 3H) ppm.
**¹³C-NMR** (101 MHz, D₂O pH* = 0) δ = 32.9, 43.7, 47.7, 59.6, 59.7, 66.1, 66.2, 171.0, 176.9 ppm.
**Anal.** Calcd (%) for C₁₄H₂₅N₃O₉·3HCl·2.5H₂O (533.78): C, 31.50; H, 6.23; N, 7.87. Found: C, 31.72; H, 6.06; N, 7.90.

### Example 5c

### Bis[µ₃-3,3'-({[1R-(1α,2α,3α,4α,5α,6α)]-5-[(carboxylato-3κO-methyl)amino-3κN⁵]-2,4,6-trihydroxylato-1κ²O²O⁶,2κ²O²O⁴,3κ²O⁴O⁶-cyclohexane-1,3-diyl}diimino-1κN¹, 2κN³)-dipropanoato-1 κO,2κO']trizirconium (IV) [Zr₃(H₋₃tacidpma)₂]

The pH of a solution of 3,3'-({[1*R*-(1α,2α,3α,4α,5α,6α)]-5-[(carboxymethyl)amino]-2,4,6-trihydroxy cyclohexane-1,3-diyl}diimino)dipropanoic acid trihydrochloride (300 mg, 0.62 mmol) and Zirconium(IV)chloride (215 mg, 0.92 mmol) in water (7 mL) was adjusted to 1.0 by addition of aqueous ammonia (16%) and water was added to reach a total volume of 16 mL. The vessels were sealed and irradiated in a microwave reactor for 120 minutes at 140°C. After cooling to room temperature the solution was adjusted to 6.5 by addition of aqueous ammonia. The turbid reaction mixture was filtrated, diluted to a volume of 100 mL and an ultrafiltration of the filtrate through a 500 da membrane (Millipore YC05) was repeated 3 times by addition of desalted water. The retentate was collected, diluted to a total volume of 500 mL and passed through a 3000 da ultrafiltration membrane (Millipore Ultracel® 3kDa) while dilution of the retentate was repeated two times. The combined 3000 da filtrates were concentrated by ultrafiltration through a 500 da membrane to a final volume of 100 mL and lyophilized to yield 194 mg of the title compound [Zr₃(H₋₃tacidpma)₂].

**¹H-NMR** (600 MHz, D₂O): δ = 2.50 - 2.70 (m, 8H), 3.01 - 3.14 (m, 4H), 3.18 - 3.33 (m, 4H), 3.40 - 3.57 (m, 4H), 3.57 - 3.72 (m, 2H), 3.84 - 3.98 (m, 2H), 3.98 - 4.18 (m, 2H), 4.77-4.95 (m, 3H), 5.01 - 5.19 (m, 4H), 5.21 - 5.37 (m, 1 H) ppm.
**MS** (ES⁺): *m*/*z* = 510.9 ([Zr₃(H₋₃tacidpma)₂]+2H)²⁺, 1020.6 ([Zr₃(H₋₃tacidpma)₂]+H)⁺.
**MS** (ES-): *mlz*= 1019.3 {[Zr₃(H₋₃tacidamp)₂]-H)⁻.
**UPLC** (ACN-HCOOH polar): Rt. = 0.22 min.

### Example 6 [Zr₃(H₋₃tacidamp)₂]

### Bis[µ₃-2,2'-({[1R-(1α,2α,3α,4α,5α,6α)]-5-[(2-carboxylato-3κO-ethyl)amino-3κN⁵]-2,4,6-trihydroxylato-1κ²O²O⁶,2κ²O²O⁴,3κ²O⁴O⁶-cyclohexane-1,3-diyl}diimino-1κN¹,2N³)di-acetato-1κO,2κO']trizirconium(IV)

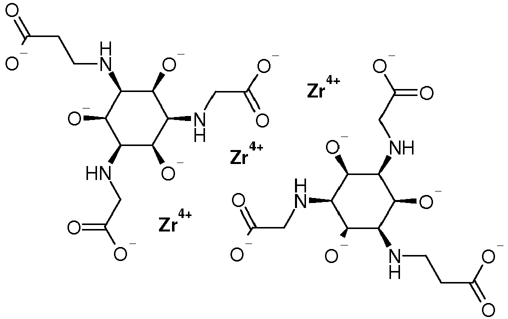

### Example 6a

### Di-tert-butyl 2,2'-({[1R-(1α,2α,3α,4α,5α,6α)]-2,4,6-tris[benzyloxy]-5-[(2-cyanoethyl)-amino]cyclohexane-1,3-diyl}diimino)diacetate

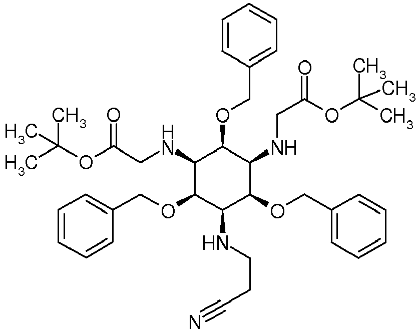

Tbcada (500 mg, 0.74 mmol) was dissolved in methanol (50 mL) and acrylonitrile (0.24 mL, 3.7 mmol) was added. The solution was stirred for 24 hours at ambient temperature. The solvent was removed, resolved in methanol, which was removed again. The residue was purified by chromatography on amino phase silica gel (ethyl acetate in hexane, 20 to 100%) to yield 447 mg of the title compound.

**¹H-NMR** (300 MHz, DMSO-*d6*): δ = 1.32 (s, 18H), 2.92 (t, 2H), 3.28 (m, 6H), 3.42 (s, 6H), 4.48 - 4.61 (m, 6H), 7.21 - 7.39 (m, 15H) ppm.

### Example 6b

### 2,2'-({[1R-(1α,2α,3α,4α,5α,6α)]-5-[(2-Carboxyethyl)amino]-2,4,6-trihydroxycyclohexane-1,3-diyl}diimino)diacetic acid trihydrochloride (H₅tacidampCl₃)

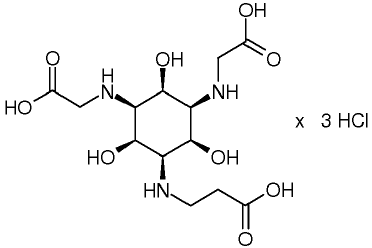

Di-*tert*-butyl 2,2'-({[1*R*-(1α,2α,3α,4α,5α,6α)]-2,4,6-tris[benzyloxy]-5-[(2-cyanoethyl)amino] cyclohexane-1,3-diyl}diimino)diacetate (341 mg, 0.47 mmol) was suspended in hydrochloric acid (6 M, 15 mL) and heated to reflux for 3 hours. After cooling, the solution was evaporated to dryness, the remaining solid dissolved in water and sorbed on DOWEX 50W-X2. The column was washed with water (0.5 L) and 0.5 M hydrochloric acid (0.5 L) and the product was eluted with 3 M hydrochloric acid (0.5 L). The eluant was removed and the solid dried in vacuo to yield 170 mg of the title compound H₃tacidamp·3HCl.

### ¹H-NMR (300 MHz, D₂O): δ = 2.88 (t, 2H), 3.46 (t, 2H), 3.62 (t, 1 H), 3.67 (t, 2H), 3.94 (s, 4H), 4.64 (t, 3H) ppm.

### Example 6c

### Bis[µ₃-2,2'-({[1R-(1α,2α,3α,4α,5α,6α)]-5-[(2-carboxylato-3κO-ethyl)amino-3κN⁵]-2,4,6-trihydroxylato-1κ²O²O⁶,2κ²O²O⁴,3κ²O⁴O⁶-cyclohexane-1,3-diyl}diimino-1 κN¹,2κN³)di-acetato-1κO,2κO']trizirconium(IV) [Zr₃(H₋₃tacidamp)₂]

A solution 2,2'-({[1*R*-(1a,2α,3α,4α,5α,6α)]-5-[(2-carboxyethyl)amino]-2,4,6-trihydroxycyclo-hexane-1,3-diyl}diimino)diacetic acid trihydrochloride (1.5g, 3.16mmol) and Zirconium(IV)chloride (1.1 g, 4.74 mmol) in water (35 mL) was adjusted to a pH of 1.0 by addition of aqueous ammonia (16%) and water was added to reach a total volume of 80 mL. The pressure vessel was sealed and heated for 120 minutes at 150°C, while 30 minutes were use as temperature ramp. After cooling to room temperature the solution was adjusted to pH 6.5 by addition of aqueous ammonia (16%). The turbid reaction mixture was filtrated and an ultrafiltration of the still turbid filtrate through a 500 da membrane (Millipore YC05) was repeated 5 times by addition of desalted water. The retentate was collected, diluted to a total volume of 500 mL and passed through a 3000 da ultrafiltration membrane (Millipore Ultracel® 3kDa) while dilution of the retentate was repeated two times. The combined 3000 da filtrates were concentrated by ultrafiltration through a 500 da membrane to a final volume of 100 mL which was lyophilized to yielded 0.97 g of the title compound [Zr₃(H₋₃tacidamp)₂].

**¹H-NMR** (600 MHz, D₂O): δ = 2.54 - 2.71 (m, 4H), 3.03 - 3.15 (m, 2H), 3.19 - 3.34 (m, 2H), 3.44 - 3.60 (m, 2H), 3.61 - 3.77 (m, 4H), 3.84 - 3.97 (m, 4H), 3.99 - 4.19 (m, 4H), 4.65-4.87 (m, 5H), 4.91 - 4.99 (m, 1 H), 4.99 - 5.05 (m, 1 H), 5.06 - 5.19 (m, 2H), 5.28 (br., 1 H) ppm.
**MS** (ES⁺): *m*/*z* = 497.8 {[Zr₃(H₋₃tacidamp)₂]+2H}²⁺, 993.6 {[Zr₃(H₋₃tacidamp)₂]+H]⁺.
**MS** (ES-): *m*/*z* = 990.8 {[Zr₃(H₋₃tacidamph]-H}⁻.
**UPLC** (ACN-HCOOH polar): Rt. = 0.22 min.

### Example 7

### Stability of bis azainositol Zirconium complexes

The stability of bis azainositol Zirconium complexes was determined in aqueous, buffered solution at pH 7.4. The solution containing 5 mmol/L of the compound in a tightly sealed vessel was heated to 121 °C for 45 minutes in a steam autoclave. The Zirconium concentration of the solution was determined by ICP-OES before and after heat treatment. The integrity of the compound was determined by HPLC analysis before and after heat treatment. Absolute stability was calculated as the ratio of the peak area of the compound after and before the heat treatment multiplied with the ratio of the metal concentration of the solution after and before heat treatment.

### HPLC system:

Column 1: Reversed phase C18.
Column 2: ZIC^{®}-HILIC.
Solvent A1: 0,5 mM tetrabutylammonium phosphate pH 6
Solvent A2: 0.1 % formic acid in water
Solvent B1: methanol
Solvent B2: acetonitrile

The use of columns, solvents, flow rate and gradients is detailed in the table below. Detector: element specific detection by ICP-MS, running at m/z 90, the most abundant isotope of Zirconium.

| | | Chromatographic conditions | | | | |
|---|---|---|---|---|---|---|
| Example No | Stability | Column | Solvent A | Solvent B | Gradient | Flow |
| 1 | | | | | | |
| 2 | | | | | | |
| 3 | 100% | 2 | A2 | B2 | 60 -15% B2 in 10 min | 1 mL/min |
| 4 | 100% | 2 | A2 | B2 | 60 -15% B2 in 10 min | 1 mL/min |
| 5 | | | | | | |
| 6 | | | | | | |

## Claims

1. Trinuclear Zirconium complexes of general formula (I), 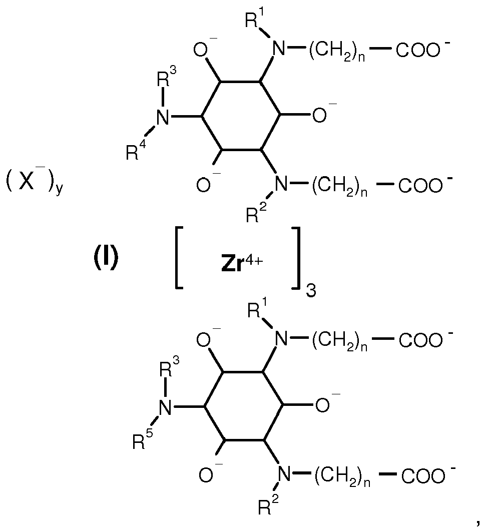 wherein
the substituents at the cyclohexyl rings exhibit an all-cis configuration;
R¹ , R² and R³ are independently from each other H or methyl;
R⁴ and R⁵ are independently from each other H, CH₃, CH₂CH₃, CH₂CH₂CH₃, CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), CH(CH₂OH)(CH₂COO⁻); CH(CH₂CH₂OH)(COO⁻) or CH(CH₂OH)(COO⁻);
m is 1 or 2;
n is 1 or 2;
y is 0, 1 or 2;
and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

2. The compound according to claim 1,
wherein
the substituents at the cyclohexyl rings exhibit an all-cis configuration;
R¹ , R² and R³ are independently from each other H or methyl;
R⁴ and R⁵ are independently from each other H, CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOO⁻, CH(CH₂OH)(CH(OH)CH₂OH), CH(CH₂OH)(CH₂COO⁻), CH(CH₂CH₂OH)(COO⁻) or CH(CH₂OH)(COO⁻);
m is 1 or 2;
n is 1 or 2;
y is 0, 1 or 2;
and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

3. The compound according to claim 1 or 2,
wherein
the substituents at the cyclohexyl rings exhibit an all-cis configuration;
R¹ , R² and R³ are independently from each other H or methyl;
R⁴ and R⁵ are independently from each other H, CH₂CH₂OH, CH₂CH₂CH₂OH, CH₂CH(OH)CH₂OH, CH₂CH(OH)CH(OH)CH₂OH, or (CH₂)ₘCOO⁻;
m is 1 or 2;
n is 1 or 2;
y is 0, 1 or 2;
and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

4. The compound according to any of the claims 1 , 2 or 3, wherein
wherein
the substituents at the cyclohexyl rings exhibit an all-cis configuration;
R¹ , R² and R³ are independently from each other H or methyl;
R⁴ and R⁵ are independently from each other H, or (CH₂)ₘCOO⁻;
m is 1 or 2;
n is 1 or 2;
y is 0, 1 or 2;
and
X⁻ is OH⁻ or Cl⁻;
or a protonated species, a deprotonated species, a stereoisomer, a tautomer, a hydrate, a solvate, or a pharmaceutically acceptable salt thereof, or a mixture of same.

5. The compound according to any of the claims 1 to 4, which is selected from the group consisting of :
Bis[µ₃-2,2',2"-({[1*R*-(1α,2α,3α,4α,5α,6α)]-2,4,6-trihydroxylato-1κ²*O*²*O*⁶,2κ²*O*²*O*⁴,3κ²*O*⁴*O*⁶-cyclohexane-1,3,5-triyl}triimino-1κ*N*¹,2κ*N*³,3*N*⁵)triacetato-1κ*O*,2κ*O*',3*O*"]trizirconium(IV) ;
Bis[µ₃-3,3',3"-({[1*R*-(1α,2α,3α,4α,5α,6α)]-2,4,6-trihydroxylato-1κ²*O*²*O*⁶,2κ²*O*²*O*⁴,3κ²-*O*⁴*O*⁶-cyclohexane-1,3,5-triyl}triimino-1κ*N*¹,2κ*N*³,3κ*N*⁵)tripropanoato-κ*O*,2κ*O*,3κ*O"* ] trizirconium(IV) ;
Bis[µ₃-3,3',3"-({[1*R*-(1α,2α,3α,4α,5α,6α)]-2,4,6-trihydroxylato-1κ²*O*²*O*⁶, 2κ²*O*²*O*⁴,3κ²*O*⁴*O*⁶-cyclohexane-1,3,5-triyl}tris{methylimino-1κ*N*¹,2κ*N*³,3κ*N*⁵})tripropanoato-κ*O*,2κ*O*',3κ*O*"] trizirconium(IV) ;
Hydroxido-3κ*O*-[µ₃-3,3',3"-({[1*R*-(1α,2α,3α,4α,5α,6α)]-2,4,6-trihydroxylato-1κ²*O*²*O*²,2κ²*O*²*O*⁴,3κ²*O*⁴*O*⁶-cyclohexane-1,3,5-triyl}tris{methylimino-1κN¹,2κ*N*³,3κ*N*⁵}) tripropanoato-1κ*O*,2κ*O*',3κ*O"*] [µ₃-3,3'-({[1*R*-(1 α,2α,3α,4α,5α,6α)]-2,4,6-trihydroxylato-1κ²*O*²*O*⁶,2κ²*O*²*O*⁴,3κ²*O*⁴*O*⁶-5*-*[methylamino-3κ*N*⁵]cyclohexane-1,3-diyl}bis{methylimino-1κ*N*¹,2κ*N*³})dipropanoato-1κ*O*,2κ*O*']trizirconium(IV) ;
Bis[µ₃-3,3'-({[1*R*-(1α,2α,3α,4α,5α,6α)]-5-[(carboxylato-3κ*O*-methyl)amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³) dipropanoato-1κ*O*,2κ*O*]trizirconium (IV) ; and
Bis[µ₃-2,2'-({[1*R*-(1α,2α,3α,4α,5α,6α)]-5-[(2-carboxylato-3κ*O*-ethyl)amino-3κ*N*⁵]-2,4,6-trihydroxylato-1κ²*O*²*O*⁶,2κ²*O*²*O*⁴,3κ²*O*⁴*O*⁶-cyclohexane-1,3-diyl}diimino-1κ*N*¹,2κ*N*³) diacetato-1κ*O*,2κ*O*]trizirconium(IV).

6. A method of preparing a compound of general formula (I) according to any one of claims 1 to 5, said method comprising the step of allowing an intermediate compound of general formula (II) 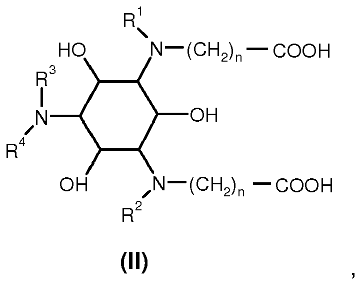 in which
the substituents at the cyclohexyl ring exhibit an all-cis configuration;
R¹ , R² and R³ are independently from each other H or methyl;
R⁴ is H, CH₃, CH₂CH₃, CH₂CH₂CH₃, CH₂CH₂OH, CH₂CH₂CH₂OH, CH₂CH(OH)CH₂OH, CH₂CH(OH)CH(OH)CH₂OH, CH(CH₂OH)₂, (CH₂)ₘCOOH, CH(CH₂OH)(CH(OH)CH₂OH), CH(CH₂OH)(CH₂COOH), CH(CH₂CH₂OH)(COOH), CH(CH₂OH)(COOH);
m is 1 or 2;
n is 1 or 2;
or a protonated species, a deprotonated species, a hydrate, a solvate, or a salt thereof, to react with
a Zirconium (IV) halogenide,
in which
halogenide is either chloride or bromide,
or hydrates thereof,
or, to react with
Zirconium(IV) oxychloride,
or hydrates thereof,
thereby giving a compound of general formula (I) 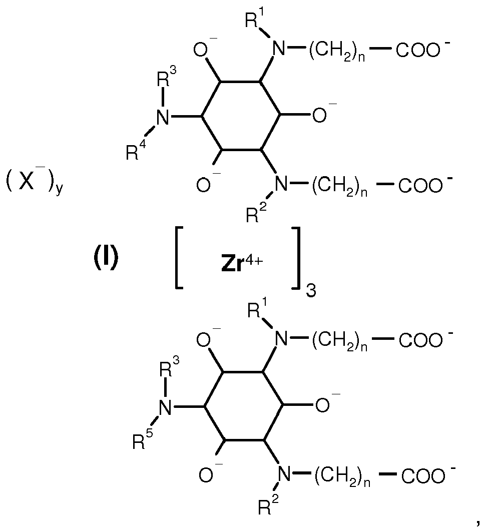 in which the substituents at the cyclohexyl rings exhibit an all-cis configuration, and in which R¹, R², R³, R⁴, R⁵, m, n, y and X are as defined for the compound of general formula (I) according to any one of claims 1 to 5.

7. Use of a compound of any one of claims 1 to 5 for diagnostic imaging.

8. A compound according to any one of claims 1 to 5 for use in the diagnosis of a disease.

9. A compound according to any one of claims 1 to 5 for use in the diagnosis of breast cancer.

10. Use of a compound of any one of claims 1 to 5 in the diagnosis of a disease.

11. Use of a compound of any one of claims 1 to 5 in the diagnosis of breast cancer.

12. Use of a compound of any one of claims 1 to 5 as diagnostic agent.

13. Use of a compound of any one of claims 1 to 5 as X-ray diagnostic agent.

14. A compound according to any one of claims 1 to 5 for the manufacture of diagnostic agents.
